# EUROPEAN PATENT SPECIFICATION

(11) **EP 3 185 363 B1**
(45) Date of publication and mention of the grant of the patent: **12.02.2020**
(21) Application number: 15202412.1
(22) Date of filing: 23.12.2015
(51) Int. Cl.: H01R 4/24, H01R 4/20, H01R 13/03, H01R 24/58

(54) **PROCESS FOR ELECTRICALLY CONTACTING A COATED LEAD WITH A LAYER**
VERFAHREN ZUR ELEKTRISCHEN KONTAKTIERUNG EINER BESCHICHTETEN LEITUNG MIT EINER SCHICHT
PROCÉDÉ DE MISE EN CONTACT ÉLECTRIQUE D'UN CONDUCTEUR REVÊTU D'UNE COUCHE

(43) Date of publication of application: 28.06.2017
(73) Proprietor: Heraeus Deutschland GmbH & Co. KG, 63450 Hanau (DE)
(72) Inventor: Keitel, Oliver, 63741 Aschaffenburg (DE); Trötzschel, Jens, 63549 Ronneburg (DE); Schibli, Stefan, 60326 Frankfurt (DE)
(74) Representative: Herzog IP Patentanwalts GmbH

(56) References cited:
- EP-A1- 2 789 366
- US-A1- 2007 239 245

## Description

### Technical Field

In general, the present invention relates generally to a process for electrically contacting a coated lead. More specifically, the invention relates to a process, an electrically contacted lead and a medical device comprising an electrically contacted lead.

### Background

Making electrical contact with an electrically conductive lead can often represent a significant challenge. In order to take advantage of beneficial properties of an electrical lead, such as good electrical conductivity and good durability, the contact to the lead itself must also display similar beneficial properties. Such considerations are particularly pertinent for example when electrically contacting leads in medical devices. In devices which are introduced into the body, it is desirable to employ thin leads, which can represent a challenge to electrically contact due their size. Furthermore, a very high value is placed on reliability in medical devices such as Cardiac Pacemakers, Implantable Cardioverter Defibrillation Devices and Cardiac Resynchronisation Devices, especially in terms of resistance to physical fatigue. Invasive surgery is required to implant medical devices into the body or remove or replace parts, and it is highly desirable for the individual components of the device to have a long working life in order to reduce the requirement for surgical intervention. Furthermore, it is desirable for the working life to have a low variance. One component of a medical device which is exposed to a particularly high amount of stress during normal operation is the lead and the electrical connections thereto.

Document US 7,364,479 B1 discloses a method for contacting a lead by crimping. Direct crimping can result in a contact which is lost over time due to physical movement. Furthermore, the direct crimping method is not suitable for multi-core leads since contact would be made to the cores indiscriminately.

Document US 2013/0338745 A1 discloses a method for contacting individual conductive cores of a cable which comprises multiple conductive cores. The individually coated conductive cores are fed through the lumens of a ring, and electrical contact is made with the individual cores by piercing. This method suffers at least from the disadvantages associated with moveable parts.

Document EP 2 789 366 A1 discloses an approach to producing a high density electrode structure having a coated lead comprising:
a. an electrically conductive core
b. an electrically insulating coating which coats the electrically conductive core
c. a via hole in the electrically insulating coating which exposes a section of the electrically conductive core
d. a first electrically conductive material which contacts at least a part of the exposed section of the electrically conductive core via the via hole
e. a further electrically conductive material which contacts at least a part of the first conductive material.

Document US 2007/239245 A1 discloses a coating for a cardiac lead.

### Summary of the invention

The invention is generally based on the object of overcoming at least one of the problems encountered in the state of the art in relation to electrical contacts with leads.

More specifically, the invention is based on the object of providing a process for electrically contacting a lead, in particular which provides for one or more selected from the following group of advantages: an improved electrical contact, an improved durability and a more flexible process.

One object of the invention is to provide a process which is suitable for electrically contacting a thin lead, preferably whilst providing for one or more selected from the following group of advantages: an improved electrical contact, an improved durability and a more flexible process.

Another object of the invention is to provide a process which is suitable for electrically contacting a lead for use in a medical device, in particular a thin lead. In particular, it is preferred for electrically contacting a lead in a bio-compatible device, preferably whilst providing for one or more selected from the following group of advantages: an improved electrical contact, an improved durability and a more flexible process. One aspect of this object is to provide a process for connecting a lead which fulfils the requirements laid out in the "Guidance for the Submission of Research and Marketing Applications for Permanent Pacemaker Leads and for Pacemaker Lead Adaptor 510(k) Submissions" issued by the "Center for Devices and Radiological Health" of the US Food and Drug Administration. A further object is provide a method for electrically contacting a multi-core lead, in particular for discriminately contacting a single core thereof. This has long been attempted and there are various approaches provided in the art. Some of these have been discussed above. There remains, however, a need for further improvement of such electrical contacts and methods for making them.

### Detailed description of the invention

A contribution to achieving at least one of the above described objects is made by the subject matter of the category forming claims of the invention. A further contribution is made by the subject matter of the dependent claims of the invention which represent specific embodiments of the invention.

### Process for electrically contacting a lead

A contribution to achieving at least one of the above mentioned objects is made by a process for electrically contacting a lead. The process according to the invention comprises the following steps:
a. Providing a coated lead comprising an electrically conductive core and an electrically insulating coating;
b. Providing a via hole in the electrically insulating coating in order to expose a section of the electrically conductive core;
c. Applying a first electrically conductive material to the coated lead such that it contacts at least a part of the exposed section of the electrically conductive core via the via hole;
d. Applying a further electrically conductive material to the coated lead such that it contacts at least a part of the first conductive material;
wherein the first electrically conductive material comprises a polymer.

In one embodiment, the lead is electrically contacted one time by the process according to the invention. In another embodiment, the lead is electrically contacted two or more times by the process according to the invention.

The lead of step a. is the lead to be contacted. The conductive core is preferably longitudinal, extending along the lead. The electrically insulating coating preferably extends along the length of the lead, coating the electrically conductive core. The process provides points of electrical contact at two points along the lead such that in use the lead provides an electrical connection between the points of electrical contact.

The via hole of step b. is provided in the electrically insulating coating order to expose a section of the electrically conductive core. The via preferably does not extend to a significant extent into the electrically conductive core, preferably not more than about 10 µm, more preferably not more than about 1 µm, more preferably still not more than about 0.1 µm, most preferably the via hole does not extend into the electrically conductive core. The section of the electrically conductive core is preferably exposed in order to allow electrical connection thereto. The area of the electrically conductive core exposed is preferably sufficient to allow electrical contact.

The via hole may be provided mechanically or otherwise. Preferred methods of providing the via hole are cutting or laser ablation, preferably laser ablation.

The first electrically conductive material of step c. is applied to the coated lead in order to make contact with the electrically conductive core via the via hole. Preferably, at least a part of the first electrically conductive material occupies at least a part of the volume of the via hole. The first electrically conductive material preferably spans the depth of the via hole in order to allow contact with the further electrically conductive material.

The thickness of the first electrically conductive material is preferably the radial distance from the surface of the electrically conductive core to the surface of the first electrically conductive material radially most distant from the electrically conductive core. In one embodiment, the first electrically conductive material is present as a layer and the thickness is the thickness of the layer. It is preferred for the first electrically conductive material to have a thickness which is not significantly less than the depth of the via hole. It is preferred for the thickness of the first electrically conductive material to be sufficient for contact to be made with the further electrically conductive material applied thereto, which is preferably applied mechanically thereto, either with no deformation or with slight deformation of the insulating coating. In one aspect of this embodiment, the thickness of the first electrically conductive material is 0.8 times or more, preferably 0.9 time or more, more preferably 1 time or more the thickness of the insulating coating. In another aspect of this embodiment, the thickness of the first electrically conductive material is greater than the thickness of the insulating coating, preferably at least more than 1, preferably at least about 1.3 times, more preferably at least about 1.5 times the thickness of the insulating coating.

The further electrically conductive material of the step d. is provided in contact with the first electrically conductive material in order to provide an electrical contact via the first electrically conductive material to the electrically conductive core. The further electrically conductive material preferably provides mechanical stability to the contact with the electrically conducting core.

It is preferred for the further electrically conductive material to be applied by solid deformation, preferably comprising one or more selected from the list consisting of: crimping, notching, bending, twisting, screwing and press fitting, preferably crimping.

### Coated lead

The coated lead preferably has a core/coating structure. The coated lead preferably has a longitudinal core extending along the lead and a coating which surrounds the core.

In one embodiment the electrically conductive core has a circular cross section. A circular cross section is preferably one which increases the ratio of cross sectional area to cross sectional circumference. In one embodiment the cross sectional area (CSA) divided by the square of the cross sectional circumference (CSC), expressed in the form (CSA)/(CSC)², is at least about 0.06, preferably at least about 0.07, more preferably at least about 0.075, most preferably at least about 0.077.

In one embodiment, the insulating coating has a thickness which is less than the radius of the electrically conductive core, preferably having a thickness which is in the range from about 0.01 to about 1 times, preferably in the range from about 0.1 to about 0.95 times, more preferably in the range from about 0.3 to about 0.9 times the radius of the electrically conductive core.

In one embodiment, the electrically conductive core preferably constitutes the major part of the coated lead, preferably by mass or by thickness or both.

In one embodiment, the cross sectional diameter of the lead is in the range from about 0.05 to about 2 mm, preferably in the range from about 0.1 to about 1 mm, more preferably in the range from about 0.2 to about 0.8 mm, most preferably in the range from about 0.3 to about 0.7 mm.

### Electrically conductive core

The electrically conductive core is responsible for electrical conductivity along the lead. The electrically conductive core is preferably of an electrically conductive material, preferably a metallic conductor. The electrically conductive core is preferably a metal. Preferred metals in this context are one or more selected form the group consisting of: an elemental metal and an alloy. Preferred metallic elements in this context are one or more selected from the group consisting of: Pt, Ir, Ta, Pd, Ti, Fe, Au, Mb, Nb, W, Ni and Ti. Preferred alloys in this context comprise one or more selected from the group consisting of: Pt, Ir, Ta, Pd, Ti, Fe, Au, Mb, Nb, W, Ni and Ti. Preferred alloys in this context are one or more selected from the group consisting of MP35, steel, and platinum/iridium alloy. The preferred steel is stainless steel, preferably a stainless steel suitable for biological applications, preferably 316L, 301 or 304, more preferably 316L. Preferred platimum/iridium alloys have a weight ratio of Pt:Ir in the range from about 60:40 to about 98:2, preferably in the range from about 70:30 to about 95:5, more preferably in the range from about 80:20 to about 90:10.

In one embodiment, the electrically conductive core has a diameter in the range from about 0.01 to about 3 mm, preferably in the range from about 0.013 to about 1.5 mm, more preferably in the range from about 0.02 to about 1 mm, most preferably in the range from about 0.05 to about 0.7 mm.

In one embodiment the electrically conductive core is formed of two or more electroconductive strands, preferably 4 to 9 electrically conductive strands, wherein the strands are in electrical contact.

### Electrically insulating coating

The purpose of the electrically insulating coating is to provide an electrical insulation between the electrically conductive core and the surroundings. The insulating coating is preferably of an electrically insulating material, preferably a polymer. Preferred polymers are one or more selected from the group consisting of: a poly epoxide, a poly alkene, a polyester and a poly silicon. Preferred polymers are one or more selected from the group consisting of: epoxy resin, silicone, polyurethane, polystyrene, polyethylene, polyethylene terephthalate, polytetrafluoroethylene and ethylene tetra fluoro ethylene. Other polymers which provide an insulating layer may be employed as the insulating coating.

In one embodiment, the electrically insulating coating comprises two or more layers of insulating coating, preferably two or more layers of polymer. In one aspect of this embodiment, the insulating coating comprises two layers of different electrically insulating material.

The thickness of the electrically insulating coating is preferably in the range from about 3 to about 150 µm, more preferably in the range from about 5 to about 100 µm, further more preferably in the range from about 9 to about 70 µm, most preferably in the range from about 12 to about 40 µm.

### First electrically conductive material

The first electrically conductive material provided electrical contact between the electrically conductive core of the lead and the further electrically conductive material. The first electrically conductive material preferably exhibits a high electrical conductivity.

In one embodiment, the first electrically conductive material is in the form of a layer, preferably with a thickness less than about 0.5 mm, more preferably less than about 0.3 mm, further more preferably less than about 0.1 mm.

In one embodiment, the first electrically conductive material comprises a conductive polymer, preferably having a specific conductivity greater than about 10 S/cm, more preferably greater than about 100 S/cm, most preferably greater than about 500 S/cm. In some cases the specific conductivity of the conductive polymer can be as high as about 2000 S/cm or less. Preferred conductive polymers in this context comprise an alkene group, an aromatic group or both, wherein the conductive polymer comprises two or more of such groups in conjugation. These molecules preferably contribute the polymeric cation of the conductive polymer. Preferred conductive polymers comprising an aromatic ring are one or more selected from: a poly(fluorene), a polyphenylene, a polypyrene, a polyazulene, a polynaphthalene, a polypyrrole, a polycarbazole, a polyindole, a polyazepine, a polyaniline, a polythiophene, a poly(3,4-ethylenedioxythiophene) and a poly(p-phenylene sulfide), preferably a poly(3,4-ethylenedioxythiophene). Preferred conductive polymers comprising an alkene group are polyacetylenes. Preferred conductive polymers comprising both an aromatic group and an alkene group are poly(p-phenylene vinylene)s. Copolymers of two or more of the above polymers are also envisaged. Conductive polymers could, according to one embodiment, comprise monomeric anions, and according to another embodiment comprise polymeric anions. These anions are organic anions and can be of any nature. It is, however, according to one aspect of the invention preferred that these organic anions comprise an aromatic moiety. A preferred monomeric organic anion is derived from toluene sulphonic acid. A preferred polymeric organic anion is derived from polyaryl sulphonic acid, preferably polystyrene sulphonic acid, which is commonly abbreviated as PSS. The preferred conductive polymer is PEDOT or PEDOT:PSS, all commercially available from Heraeus Deutschland GmbH & Co. KG under the Trademark Clevios®. In one aspect of this embodiment, the first electrically conductive material is the conductive polymer.

In one embodiment, the first electrically conductive material comprises an electrically conductive particle. Preferred electrically conductive particles in this context are one or more selected from the group consisting of: a conductive carbon particle and a metal particle. Preferred conductive carbon particles in this context are one or more selected from the group consisting of: a carbon nanotube, a carbon nanorod, a carbon nanowire, a carbon nanosheet, a carbon nanoshell and a carbon nanoparticle; wherein carbon nano-tubes are particularly preferred. Preferred metal particles in this context are one or more selected form the group consisting of: an elemental metal and an alloy. Preferred metallic elements in this context are one or more selected from the group consisting of: Ag, Cu, Pt, Ir, Ta, Pd, Ti, Fe, Au, Mb, Nb, W, Ni and Ti, preferably Ag, Au or Cu, more preferably Ag or Au. Preferred alloys in this context comprise one or more selected from the group consisting of: Ag, Pt, Ir, Ta, Pd, Ti, Fe, Au, Mb, Nb, W, Ni and Ti. Preferred alloys in this context are one or more selected from the group consisting of MP35, steel, and platinum/iridium alloy. The preferred steel is stainless steel, preferably a stainless steel suitable for biological applications, preferably 316L, 301 or 304, more preferably 316L. Preferred platimum/iridium alloys have a weight ratio of Pt:Ir in the range from about 60:40 to about 98:2, preferably in the range from about 70:30 to about 95:5, more preferably in the range from about 80:20 to about 90:10. In one aspect of this embodiment, the conductive particles are present in a conductive polymer, preferably one of the preferred polymers described above. In another aspect of this embodiment, the conductive particles are present in a non-conductive polymer. In this context a non-conductive polymer is a polymer with a conductivity less than about 100 S/cm, or less than about 50 S/cm, or less than about 10 S/cm. Preferred non-conductive polymers are one or more selected from the group consisting of: epoxy, silicone, polyurethane, polystyrene, polyethylene terephthalate, polytetrafluoroethylene and poly (ethene-co-tetrafluoroethene); preferably selected from epoxy and silicone.

In one embodiment, the first electrically conductive material is applied as a solid, preferably as a thin solid layer. The solid is preferably wrapped around the coated lead.

In another embodiment, the first electrically conductive material is applied as a liquid which subsequently converts into a solid, preferably by curing or drying or both. In one aspect of this embodiment, the first electrically conductive material comprises two or more monomers which polymerise to yield a solid. In another aspect of this embodiment, the first electrically conductive material comprises a solvent which leaves to yield a solid.

### Second electrically conductive material

The second electrically conductive material makes electrical contact with the first electrically conductive material and in this way is in electrical contact with the electrically conductive core. The second electrically conductive material preferably exhibits metallic conductivity. The second electrically conductive material is preferably a metal. Preferred metals in this context are one or more selected form the group consisting of: an elemental metal and an alloy. Preferred metallic elements in this context are one or more selected from the group consisting of: Pt, Ir, Ta, Pd, Ti, Fe, Au, Mb, Nb, W, Ni and Ti. Preferred alloys in this context comprise one or more selected from the group consisting of: Pt, Ir, Ta, Pd, Ti, Fe, Au, Mb, Nb, W, Ni and Ti. Preferred alloys in this context are one or more selected from the group consisting of MP35, steel, and platinum/iridium alloy. The preferred steel is stainless steel, preferably a stainless steel suitable for biological applications, preferably 316L, 301 or 304, more preferably 316L. Preferred platimum/iridium alloys have a weight ratio of Pt:Ir in the range from about 60:40 to about 98:2, preferably in the range from about 70:30 to about 95:5, more preferably in the range from about 80:20 to about 90:10.

In one embodiment, the second electrically conductive material is of the same material as the electrically conductive core.

In one embodiment, the second electrically conductive material is a ring, preferably a crimpable ring.

In one embodiment, the second electrically conductive material has a mass in the range from about 0.05 mg to about 0.5 g, preferably in the range from about 0.1 mg to about 0.3 g, more preferably in the range from about 0.2 mg to about 1 mg.

### Multiple electrically conductive cores

In one embodiment, the lead comprises two or more electrically conductive cores, preferably with at least one of the electrically conductive cores, more preferably all of the electrically conductive cores being electrically insulated from the other electrically conductive core(s). The presence of more than one electrically conductive core in the lead preferably allows the lead to be used to create two or more isolated electrical circuits. In one embodiment, the lead comprises two or more electrically conductive cores, electrically insulated from each other, and two or more of the electrically conductive cores, preferably all of the electrically conductive cores, are electrically contacted by a process according to the invention. In one aspect of this embodiment, one or more of the electrically conductive cores, preferably all of the electrically conductive cores, is/are connected in two or more placed by a process according to the invention.

Where the lead comprises two or more electrically conductive cores, it is preferred for one or more, preferably all of the electrical contacts made by the process according to the invention to make contact with a single electrically conductive core.

Where more than one electrically conductive core is present, the preferred features of the electrically conductive core introduced above preferably apply to at least one of the electrically conductive cores, more preferably all of the electrically conductive cores.

In one embodiment, the coated lead comprises two or more coated wires with a core/coating structure, preferably twisted together to form a twisted lead. In one aspect of this embodiment, the ensemble of coated wires if further coated with an electrically insulating material.

### Medical devices

A contribution to achieving one or more of the above mentioned objects is made by a medical device comprising a contacted lead according to the present invention. Preferred medical devices in this context are one or more selected form the group consisting of: a pacemaker, a defibrillator, a cardo resynchronisation device, a neuro-stimulator and a measuring device.

### Figures

The invention is now further illustrated using figures which are not to be considered as limiting the scope of the invention. In brief, the figures show the following:
Figures 1a to 1d show schematically the process for electrically contacting a coated lead
Figure 2 shows schematically a process according to the invention for contacting a coated lead.
Figure 3 shows schematically a pacemaker comprising a lead electrically connected according to the invention
Figure 4 shows schematically a lead with 3 electrically conductive cores, contacted six times according to the invention

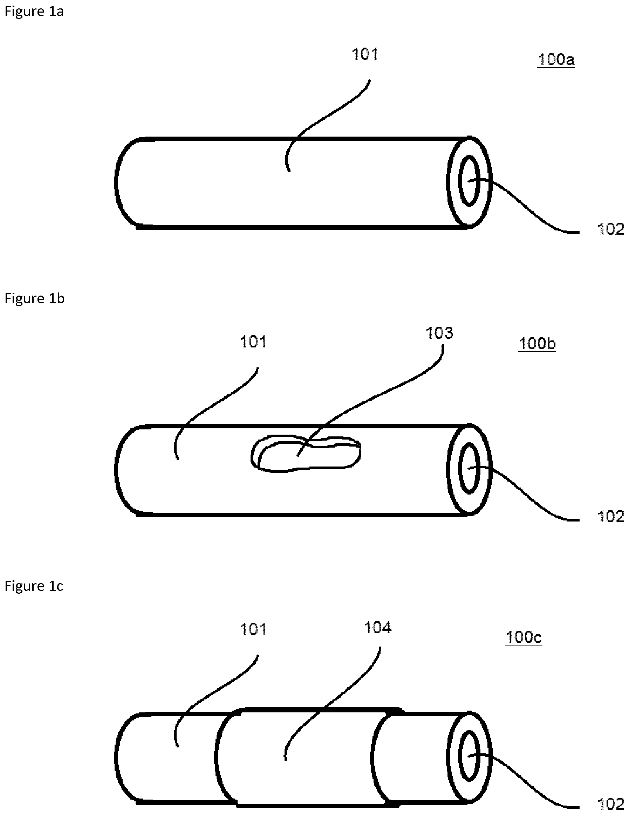

Figure 1a shows a coated lead 100a according to the invention. The coated lead 100a comprises an electrically conductive core 102 of electrically conductive material coated with an electrically insulating coating 101 of electrically insulating material. In this case, the electrically conductive material is a rhodium/platinum alloy (20 % rhodium and 80 % platinum, by mass) and the electrically conductive core has a diameter of 0.5 mm. In this case, the electrically insulating coating is 20 µm thick and the electrically insulating coating is of polyurethane.

Figure 1b shows 100b a coated lead with via hole 103. The coated lead with via hole 100b was obtained from the coated lead 100a by laser ablation. The via hole 103 extends through the electrically insulating coating 101 to expose a section of the surface of the electrically conductive core with a cross sectional area of 0.02 mm².

Figure 1c shows 100c a coated lead with a via hole 103 and a layer of electrically conductive material 104, in this case a 25 µm thick layer of PEDOT:PSS, applied over the via hole 103. The layer 104 is in electrical contact with the electrically conductive core 102. The layer 104 has been applied to the coated lead over the via hole 103 by coating.

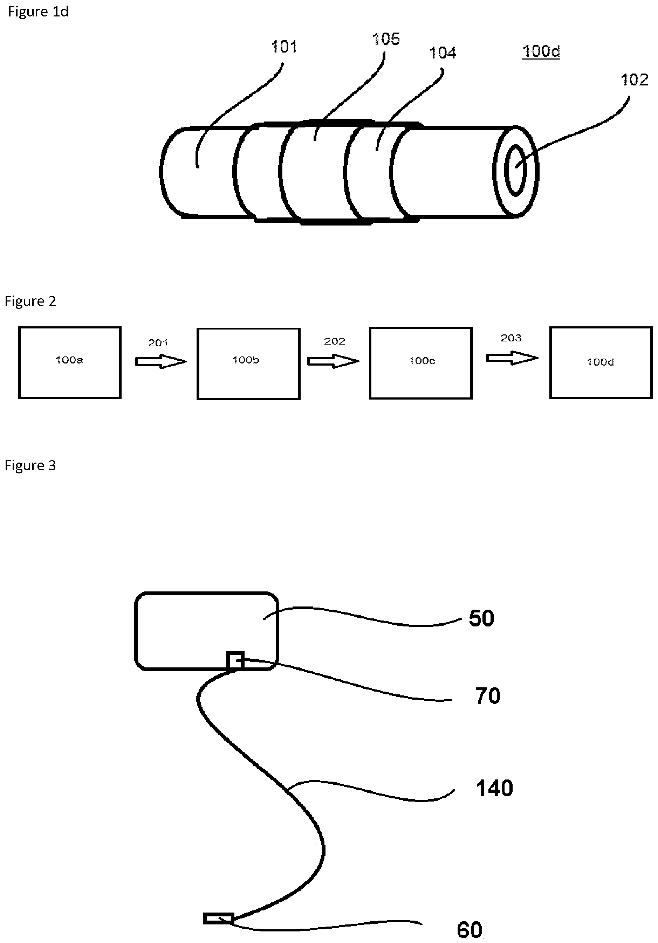

Figure 1d shows 100d a coated lead with a via hole 103, a layer of PEDOT:PSS 104 over the via hole and an electrically conductive ring 105 located over the layer 104 and the via hole 103. The conductive ring has been crimped around the lead so as to to make electrical contact with the layer 104. In this case, the conductive ring is of rhodium/platinum alloy (20 % rhodium and 80 % platinum, by mass), and has a thickness of 0.5 mm.

Figure 2 shows schematically a process according to the invention for contacting a coated lead. Figure 2 shows how the items 100a, 100b, 100c & 100d are related by process steps. In step a), a coated lead 100a is provided. In step b) 201, a via hole is provided in the insulating coating of the coated lead 100a to obtain a coated lead with a via hole 100b. In step c) 202, a layer is applied to the coated lead over the via hole to obtain a coated lead with a via hole and a layer over the via hole 100c. In step d) 203, an electrically conductive ring is crimped over the via hole and the layer to obtain the electrically contacted lead 100d.

Figure 3 shows schematically a pacemaker 50 with a pulse generator 70, and a lead 140 comprising an electrode 60. The lead 140 connects the pulse generator 70 and the heart tissue via the electrode 60. The lead 140 has been electrically connected to the pulse generator 70 and the electrode 60 by a process according to the invention.

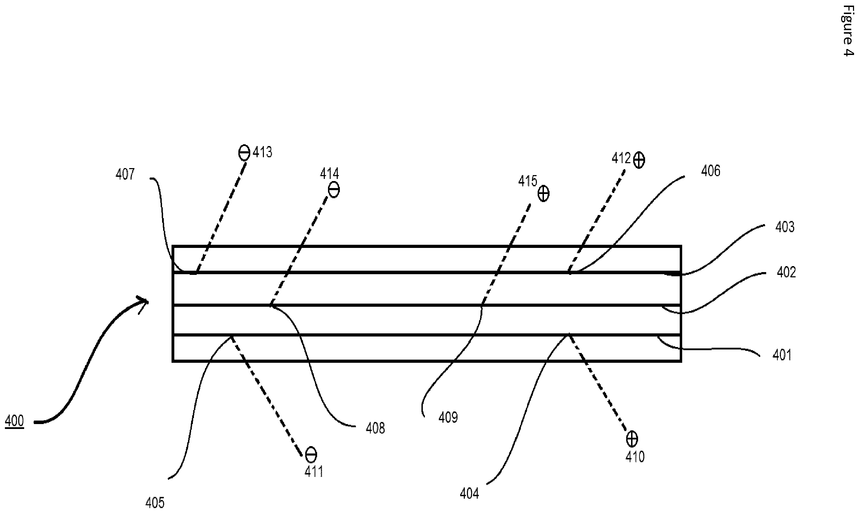

Figure 4 shows schematically a lead 400, comprising 3 electrically conductive cores 401, 402 & 403. Electrically conductive core 401 is electrically connected according to the invention at points 404 and 405 to allow an electrical circuit between notional terminals 410 and 411 respectively. Similarly electrically conductive core 402 is electrically connected according to the invention at points 408 and 409 to allow an electrical circuit between notional terminals 414 and 415 respectively. Similarly electrically conductive core 403 is electrically connected according to the invention at points 406 and 407 to allow an electrical circuit between notional terminals 412 and 413 respectively.

### Test Methods

### Fatigue Test

Fatigue resistance is measured according to the test described in "*prEN 45502 Parts 2 & 3 CEN*/*CENELEC, Active Implantable Medical Devices* - *Brady and Tachy Lead Tests Draft*/*Standard".*

### Examples

### Example 1 (inventive) - Contacting of a lead according to the invention

Electrical contacts were made to 15 cm long leads having a 0.2 mm diameter core of rhodium/platinum alloy (20 % rhodium, 80 % platinum, by mass) and a 20 µm coating of PTFE. 1 cm from each end of the lead, an electrical contact was made as follows: First, a via hole was made through the PTFE coating by laser ablation using a Varydisk laser available from Dausinger + Giesen GmbH. The via hole had a circular cross section of 60 µm diameter. Second, a layer of PEDOT:PSS was coated by spraying over the via hole in a cylindrical layer having a thickness of 30 µm and a cylinder length of 200 µm. The lead was heated to 100 °C for 5 hours to dry the PEDOT:PSS layer. Third, a cylindrical ring of rhodium/platinum alloy (20 % rhodium, 80 % platinum, by mass) with outer diameter of 350 µm, an inner diameter of 260 µm and a cylinder length of 500 µm, available from Heraeus Deutschland GmbH & Co. KG, was threaded over the lead, positioned so as to cover the via hole with applied PEDOT:PSS layer and crimped tight onto the lead using a multi-jaw chuck.

### Example 2a (comparative) - Contacting of a lead by soldering

The lead was provided as in example 1 and electrical contact was made 1 cm from each end of each lead as follows: First, a via hole was made through the PTFE coating by laser ablation using a Varydisk laser available from Dausinger + Giesen GmbH. The via hole had a circular cross section of 60 µm diameter. Second, a cylindrical ring as in example 1 was threaded over the lead, positioned so as to cover the via hole and a contact was made between the ring and the core of the lead by soldering with gold at 700 °C.

### Example 2b (comparative) - Contacting of a lead by soldering

The lead was provided as in example 1 and electrical contact was made 1 cm from each end of each lead as follows: First, a via hole was made through the PTFE coating by laser ablation using a Varydisk laser available from Dausinger + Giesen GmbH. The via hole had a circular cross section of 60 µm diameter. Second, a cylindrical ring as in example 1, but with a 60 µm via hole was provided. The ring was threaded over the lead and positioned such that the via in the ring was over the via in the coating of the lead. Contact was made between the ring and the core of the lead by soldering through the two superimposed via holes with gold at 700 °C.

### Example 3 (comparative) - Contacting of a lead by crimping

A lead was provided as in example 1 and electrical contact was made 1 cm from each end of each lead as follows: First, a cylindrical ring as in example 1 was threaded over the lead and positioned 1 cm from the end of the lead. Second, the ring was crimped using a multi-jaw chuck to contact the ring with the core of the lead.

### Properties of the contacted leads

For each of the examples (1, 2a, 2b & 3), 5 leads were contacted as described. Each contacted lead was tested for fatigue resistance. The success rate of contacting the leads and the results for fatigue resistance are shown in table 1.

**Table 2**

| Example | Successful contacting ? | Fatigue resistance |
|---|---|---|
| 1 | Yes, in all 5 samples | ++ |
| 2a | 2 leads contacted, 3 failed | -- |
| 2b | 3 leads contacted, 2 failed | -- |
| 3 | 4 leads contacted, 1 failed | -- |

| | | |
|---|---|---|
| ++ Very good fatigue resistance (no failures after 400,000,000 cycles), -- very poor fatigue resistance (failed after an average of less than 300,000 cycles) | | |

### REFERENCE LIST

- 100a: coated lead
- 100b: coated lead with via hole
- 100c: coated lead with via hole and layer over the via hole
- 100d: contacted lead
- 101: electrically insulating coating
- 102: electrically conductive core
- 103: via hole
- 104: electrically conductive layer
- 105: electrically conductive ring
- 201: step b) - providing a via hole
- 202: step c) - applying the first electrically conductive material to the lead over the via hole
- 203: step d) - applying the second electrically conductive material
- 50: pacemaker
- 70: pulse generator
- 140: lead
- 60: electrode

## Claims

1. A process for electrically contacting a coated lead (100a) comprising the following steps:
a. Providing a coated lead (100a) comprising an electrically conductive core (102) and an electrically insulating coating (101);
b. Providing a via hole (103) in the electrically insulating coating (101) in order to expose a section of the electrically conductive core (102);
c. Applying a first electrically conductive material (104) to the coated lead (100b) such that it contacts at least a part of the exposed section of the electrically conductive core (102) via the via hole (103);
d. Applying a further electrically conductive material (105) to the coated lead (100c) such that it contacts at least a part of the first conductive material (104),
wherein the first electrically conductive material (104) comprises a polymer.

2. The process according to claim 1, wherein the first electrically conductive material (104) is one or more selected from the group consisting of: an electrically conductive polymer, a composite comprising an electrically non-conductive polymer and an electrically conductive particle, and a composite comprising an electrically conductive polymer and an electrically conductive particle.

3. The process according to any of the preceding claims, wherein the first electrically conductive material (104) comprises a polymer having two or more conjugated double bonds.

4. The process according to any of the preceding claims, wherein the first electrically conductive material (104) is applied as a layer with thickness less than about 0.3 mm.

5. The process according to any of the preceding claims, wherein the first electrically conductive material (104) is applied by one or more selected from the group consisting of: dipping, printing, spraying, injecting, painting, dropping, stamping, spilling and laying.

6. The process according to any of the preceding claims, wherein the second electrically conductive material (105) is applied by solid deformation.

7. The process according to any of the preceding claims, wherein the lead (100a) has a diameter less than about 2 mm.

8. The process according to any of the preceding claims, wherein the lead (100a) comprises 2 or more electrically conductive cores (102).

9. The process according to claim 8, wherein 2 or more of the electrically conductive cores (102) are electrically insulated from each other by an insulated material.

10. The process according to any of the preceding claims, wherein the via hole (103) exposes a single conductive core (102).

11. The process according to any of the preceding claims, wherein the provision of the via hole (103) in step b. is performed with a laser.

12. An electrically contacted lead (100d) comprising the following:
a. an electrically conductive core (102)
b. an electrically insulating coating (101) which coats the electrically conductive core (102);
c. a via hole (103) in the electrically insulating coating (101) which exposes a section of the electrically conductive core (102);
d. a first electrically conductive material (104) which contacts at least a part of the exposed section of the electrically conductive core (102) via the via hole (103);
e. a further electrically conductive material (105) which contacts at least a part of the first conductive material (104),
wherein the first electrically conductive material (104) comprises a polymer.

13. A medical device (50) comprising a contacted lead (140) according to claim 12.

## Patentansprüche

1. Verfahren zum elektrischen Kontaktieren einer beschichteten Leitung (100a), umfassend die folgenden Schritte:
a. Bereitstellen einer beschichteten Leitung (100a) mit einem elektrisch leitfähigen Kern (102) und einer elektrisch isolierenden Beschichtung (101);
b. Bereitstellen eines Durchgangslochs (103) in der elektrisch isolierenden Beschichtung (101), um einen Abschnitt des elektrisch leitenden Kerns (102) freizulegen;
c. Aufbringen eines ersten elektrisch leitfähigen Materials (104) auf die beschichtete Leitung (100b), so dass sie mindestens einen Teil des freiliegenden Abschnitts des elektrisch leitfähigen Kerns (102) über die Durchgangsbohrung (103) kontaktiert;
d. Aufbringen eines weiteren elektrisch leitfähigen Materials (105) auf die beschichtete Leitung (100c), so dass sie mindestens einen Teil des ersten leitfähigen Materials (104) berührt,
wobei das erste elektrisch leitfähige Material (104) ein Polymer umfasst.

2. Verfahren nach Anspruch 1, worin das erste elektrisch leitfähige Material (104) ein oder mehrere ist, ausgewählt aus der Gruppe bestehend aus: einem elektrisch leitfähigen Polymer, einem Verbundstoff, der ein elektrisch nicht leitfähiges Polymer und ein elektrisch leitfähiges Partikel umfasst, und einem Verbundstoff, der ein elektrisch leitfähiges Polymer und ein elektrisch leitfähiges Partikel umfasst.

3. Verfahren nach einem der vorhergehenden Ansprüche, worin das erste elektrisch leitfähige Material (104) ein Polymer mit zwei oder mehr konjugierten Doppelbindungen umfasst.

4. Verfahren nach einem der vorhergehenden Ansprüche, wobei das erste elektrisch leitfähige Material (104) als Schicht mit einer Dicke von weniger als etwa 0,3 mm aufgebracht wird.

5. Verfahren nach einem der vorhergehenden Ansprüche, wobei das erste elektrisch leitfähige Material (104) durch ein oder mehrere aus der Gruppe bestehend aus: Tauchen, Drucken, Sprühen, Injizieren, Streichen, Fallenlassen, Stanzen, Verschütten und Verlegen aufgebracht wird.

6. Verfahren nach einem der vorhergehenden Ansprüche, wobei das zweite elektrisch leitfähige Material (105) durch feste Verformung aufgebracht wird.

7. Verfahren nach einem der vorhergehenden Ansprüche, worin die Leitung (100a) einen Durchmesser von weniger als etwa 2 mm aufweist.

8. Verfahren nach einem der vorhergehenden Ansprüche, worin die Leitung (100a) 2 oder mehr elektrisch leitfähige Kerne (102) umfasst.

9. Verfahren nach Anspruch 8, wobei 2 oder mehr der elektrisch leitenden Kerne (102) durch ein isoliertes Material elektrisch voneinander isoliert sind.

10. Verfahren nach einem der vorhergehenden Ansprüche, wobei das Durchgangsloch (103) einen einzelnen leitenden Kern (102) freilegt.

11. Verfahren nach einem der vorhergehenden Ansprüche, wobei die Bereitstellung der Durchgangsbohrung (103) in Schritt b. mit einem Laser durchgeführt wird.

12. Elektrisch kontaktierte Leitung (100d), umfassend das Folgende:
a. einen elektrisch leitfähigen Kern (102)
b. eine elektrisch isolierende Beschichtung (101), die den elektrisch leitenden Kern (102) beschichtet;
c. ein Durchgangsloch (103) in der elektrisch isolierenden Beschichtung (101), das einen Abschnitt des elektrisch leitenden Kerns (102) freilegt;
d. ein erstes elektrisch leitfähiges Material (104), das mindestens einen Teil des freiliegenden Abschnitts des elektrisch leitenden Kerns (102) über die Durchgangsbohrung (103) kontaktiert;
e. ein weiteres elektrisch leitfähiges Material (105), das mindestens einen Teil des ersten leitenden Materials (104) kontaktiert,
wobei das erste elektrisch leitfähige Material (104) ein Polymer umfasst.

13. Medizinische Vorrichtung (50) mit einer kontaktierten Leitung (140) nach Anspruch 12.

## Revendications

1. Procédé de mise en contact électrique d'un conducteur revêtu (100a) comprenant les étapes suivantes :
a. Fourniture d'un conducteur revêtu (100a) comprenant un noyau électriquement conducteur (102) et un revêtement électriquement isolant (101) ;
b. Prévoir un trou de passage (103) dans le revêtement électriquement isolant (101) afin d'exposer une section du noyau électriquement conducteur (102) ;
c. Application d'un premier matériau électriquement conducteur (104) sur le conducteur revêtu (100b) de telle sorte qu'il entre en contact avec au moins une partie de la section exposée du noyau électriquement conducteur (102) via le trou de passage (103) ;
d. Application d'un autre matériau électriquement conducteur (105) sur le conducteur revêtu (100c) de telle sorte qu'il soit en contact avec au moins une partie du premier matériau conducteur (104),
dans laquelle le premier matériau électriquement conducteur (104) comprend un polymère.

2. Procédé selon la revendication 1, dans lequel le premier matériau électriquement conducteur (104) est un ou plusieurs matériaux choisis dans le groupe constitué par : un polymère électriquement conducteur, un composite comprenant un polymère électriquement non conducteur et une particule électriquement conductrice, et un composite comprenant un polymère électriquement conducteur et une particule électriquement conductrice.

3. Procédé selon l'une quelconque des revendications précédentes, dans lequel le premier matériau électriquement conducteur (104) comprend un polymère ayant deux doubles liaisons conjuguées ou plus.

4. Procédé selon l'une quelconque des revendications précédentes, dans lequel le premier matériau électriquement conducteur (104) est appliqué sous la forme d'une couche ayant une épaisseur inférieure à environ 0,3 mm.

5. Procédé selon l'une quelconque des revendications précédentes, dans lequel le premier matériau électriquement conducteur (104) est appliqué par un ou plusieurs matériaux choisis dans le groupe consistant à : tremper, imprimer, pulvériser, injecter, peindre, faire goutter, estamper, renverser et poser.

6. Procédé selon l'une quelconque des revendications précédentes, dans lequel le deuxième matériau électriquement conducteur (105) est appliqué par déformation solide.

7. Procédé selon l'une quelconque des revendications précédentes, dans lequel le plomb (100a) a un diamètre inférieur à environ 2 mm.

8. Procédé selon l'une quelconque des revendications précédentes, dans lequel le plomb (100a) comprend 2 noyaux électriquement conducteurs (102) ou plus.

9. Procédé selon la revendication 8, dans lequel 2 ou plusieurs des noyaux électriquement conducteurs (102) sont isolés électriquement les uns des autres par un matériau isolé.

10. Procédé selon l'une quelconque des revendications précédentes, dans lequel le trou de passage (103) expose un seul noyau conducteur (102).

11. Procédé selon l'une quelconque des revendications précédentes, dans lequel la réalisation du trou de passage (103) dans l'étape b. est réalisée avec un laser.

12. Fil à contact électrique (100d) comprenant les éléments suivants :
a. un noyau électriquement conducteur (102)
b. un revêtement électriquement isolant (101) qui recouvre le noyau électriquement conducteur (102) ;
c. un trou de passage (103) dans le revêtement électriquement isolant (101) qui expose une section du noyau électriquement conducteur (102) ;
d. un premier matériau électriquement conducteur (104) qui est en contact avec au moins une partie de la section exposée du noyau électriquement conducteur (102) via le trou de passage (103) ;
e. un autre matériau électriquement conducteur (105) qui est en contact avec au moins une partie du premier matériau conducteur (104),
dans laquelle le premier matériau électriquement conducteur (104) comprend un polymère.

13. Dispositif médical (50) comprenant un fil de contact (140) selon la revendication 12.
